**Europäisches Patentamt**

**European Patent Office**

**Office européen des brevets**

(19)

(11) Veröffentlichungsnummer : **0 399 085 B1**

(12) **EUROPÄISCHE PATENTSCHRIFT**

(45) Veröffentlichungstag der Patentschrift :
**19.02.92 Patentblatt 92/08**

(21) Anmeldenummer : **89109556.4**

(22) Anmeldetag : **26.05.89**

(51) Int. Cl.⁵ : **C10K 1/10,** C07C 7/11,
C07C 7/17, B01D 53/14

(54) **Verfahren und Vorrichtung zum Auswaschen von Verunreinigungen aus Rohgasen.**

(43) Veröffentlichungstag der Anmeldung :
**28.11.90 Patentblatt 90/48**

(45) Bekanntmachung des Hinweises auf die
Patenterteilung :
**19.02.92 Patentblatt 92/08**

(84) Benannte Vertragsstaaten :
**AT BE DE ES FR GR IT NL**

(56) Entgegenhaltungen :
**BE-A- 542 041**
**DE-B- 1 201 944**
**DE-B- 2 549 399**
**GB-A- 768 345**

(73) Patentinhaber : **Linde Aktiengesellschaft**
**Abraham-Lincoln-Strasse 21**
**W-6200 Wiesbaden (DE)**

(72) Erfinder : **Sontag, Hans-Jürgen**
**Tulpenstrasse 14**
**W-8200 Möhldorf (DE)**
Erfinder : **Kunde, Frank**
**Zündterstrasse 18**
**W-8000 München 21 (DE)**
Erfinder : **Schraufstetter, Michael**
**Kurze Gasse 5**
**W-8059 Eichenried (DE)**

(74) Vertreter : **Schaefer, Gerhard, Dr.**
**Linde Aktiengesellschaft Zentrale**
**Patentabteilung**
**W-8023 Höllriegelskreuth (DE)**

## Beschreibung

Die Erfindung betrifft ein Verfahren sowie eine Vorrichtung zum Auswaschen von Verunreinigungen, wie Phosphorwasserstoff ($PH_3$), Ammoniak ($NH_3$), Schwefelwasserstoff ($H_2S$) und/oder Arsenwasserstoff ($AsH_3$) aus Rohgasen mittels Schwefelsäure in zumindest einer Waschsäule.

Solche Verunreinigungen sind beispielsweise im Koksofengas, Wassergas oder Rohacetylen enthalten und müssen vor einer Weiterverarbeitung entfernt werden. Insbesondere werden an das Rohacetylen hohe Anforderungen in Bezug auf Gasreinheit gestellt, z.B. bei einer Verwendung des Acetylens für die Flammphotometrie.

So wurde z.B. vorgeschlagen, diese Verunreinigungen durch Aufgabe geringer Mengen konzentrierter Schwefelsäure auf den Kopf eines Waschturms und Abzug einer entsprechenden Menge an Schwefelsäure vom Sumpf des Waschturms zu entfernen. Eine Wiederverwendung der konzentrierten Schwefelsäure nach externer Regenerierung, z.B. durch Spülen mit einem inerten Gas, ist möglich.

Ein anderes bekanntes Verfahren besteht beispielsweise darin, daß konzentrierte Schwefelsäure in einem Waschturm solange umgewälzt wird, bis die Säurekonzentration einen bestimmten Wert unterschreitet. Danach wird die verbrauchte Säure verworfen und neue frische Säure eingefüllt. Das Verfahren hat den Nachteil, daß nur kurz nach Einfüllung der frischen Säure eine ausreichende Gasreinheit - z.B. bei Acetylen für die Flammphotometrie - erreicht werden kann.

Das letztgenannte Verfahren hat den großen Nachteil, daß es sich um einen diskontinuierlichen Prozeß handelt, was im Gegensatz zum kontinuierlichen Rohgasanfall z.B. bei der Acetylenproduktion steht.

Ein weiteres Problem des bekannten Verfahrens besteht darin, daß die Schwefelsäure die Bildung von Polimerisationsprodukten aus dem Rohgas, wie z.B. Acetylen begünstigt. Diese Produkte haben eine dem Teer ähnliche Konsistenz und führen zu Verlegungen der Einbauten in den Waschsäulen, z.B. bei Verwendung von Füllkörpersäulen zum Verkleben der Füllkörper.

Mit zunehmender Temperatur steigt die Acetylenzersetzung stark an. Des weiteren führen Ablagerungen von Ammoniumsulfat zu Verlegungen im unteren Teil der Säule, was kurze Standzeiten der Waschtürme zur Folge hat. Aus diesem Grund sind z.B. die meisten Acetylenwerke mit zwei oder mehr Säurewaschtürmen ausgestattet, die wechselweise betrieben werden. Dies bedingt hohe Investitions- und Betriebs- bzw. Reparaturkosten.

Überdies ist es nachteilig, daß die herkömmlichen Säurewaschtürme sehr hoch sein müssen (z.B. zwischen 7 und 10 m) und entsprechend hohe Gebäude benötigen, um auf diese Weise eine hinreichende Reaktionszeit zwischen Gas und Säure zu erzielen.

Es ist daher Aufgabe der vorliegenden Erfindung, ein Verfahren sowie eine Vorrichtung der eingangs genannten Art so zu verbessern, daß auf einfache und kostengünstige Weise eine kontinuierliche Auswaschung der Verunreinigungen aus Rohgasen erreicht wird.

Diese Aufgabe wird erfindungsgemäß dadurch gelöst, daß in einer (n-2)ten Waschstufe, wobei n größer-/gleich 3 ist, verdünnte Schwefelsäure und in einer (n-1)ten Waschstufe konzentrierte Schwefelsäure im Kreislauf geführt werden sowie in einer n-ten Waschstufe frische konzentrierte Schwefelsäure am Kopf der Waschsäule aufgegeben wird.

Somit wird beispielsweise bei der dreistufigen Wäsche im ersten Waschabschnitt verdünnte Schwefelsäure umgewälzt, deren Wasseraufnahmevermögen ausreicht, um in den Rohgasen enthaltenes Wasser zu entfernen. Gleichzeitig wird in dem Rohgas vorhandener Ammoniak zu Ammonsulfat $(NH_4)_2SO_4$ umgesetzt. Das Produkt bleibt in Lösung und wird aus dem Sumpf mitabgezogen. Hieran schließt sich ein zweiter Waschabschnitt mit konzentrierter Schwefelsäure an, in dem eine Auswaschung von $PH_3$, $H_2S$ und $AsH_3$ aus dem Rohgas bewirkt wird. Dabei entstehen als Umsetzungsprodukte Phosphorsäure, $SO_2$, $As_2O_3$ und Wasser bzw. schweflige Säure, die zusammen mit der konzentrierten Schwefelsäure im Kreis geführt werden bzw. zumindest teilweise dem Rohgas und damit der ersten Waschstufe zugeführt werden.

Im dritten Waschabschnitt im Kopf der Säule wird erfindungsgemäß frische konzentrierte Schwefelsäure eindosiert, rieselt z.B. durch ein Füllkörperbett und wird anschließend dem konzentrierten Säurekreislauf der zweiten Stufe zugeführt. Da konzentrierte Schwefelsäure bei der Auswaschung von Phosphorwasserstoff hauptsächlich eine katalytische Wirkung besitzt, findet in der zweiten Waschstufe außer der Umsetzung zu Phosphorsäure auch eine Lösung von Phosphorwasserstoff in der konzentrierten Schwefelsäure statt, welcher bei der Wiedereindüsung der konzentrierten Schwefelsäure freigesetzt und in der dritten Stufe durch die herabrieselnde zudosierte Säure wieder ausgewaschen wird. Auf diese Weise wird kontinuierlich eine größtmögliche Gasreinheit (Verunreinigungen 3 - 0 ppm) gewährleistet.

Vom Sumpf der (n-2)ten Waschstufe wird erfindungsgemäß überschüssige verdünnte Schwefelsäure abgezogen, da es ansonsten durch die kontinuierliche Zudosierung von frischer konzentrierter Schwefelsäure am Kopf der n-ten Waschstufe zur Akkumulation innerhalb des Verfahrens kommen würde. Auch werden auf

2

diese Weise während der Wäsche gebildetes Ammoniumsulfat und geringe Mengen von Polymerisationsprodukten ausgeschleust.

Gegenüber dem Stand der Technik bietet die Erfindung den Vorteil, daß mit einer hohen Säureumlaufmenge (3 - 7m³) gearbeitet wird, wodurch die in geringen Mengen gebildeten Polymerisationsprodukte abgespült werden und somit einen kontinuierlichen Betrieb nicht behindern. Mit Vorteil werden dabei 5 bis 20 l Schwefelsäure pro m³ zu reinigendes Gas im Kreislauf geführt. Insgesamt wird bei dem erfindungsgemäßen Verfahren Schwefelsäure im Überschuß zugegeben, beispielsweise 2,5 l pro 100 m³/h zu reinigendes Gas.

Gemäß zweier vorteilhafter Ausgestaltungen des erfindungsgemäßen Verfahrens werden die n Waschstufen entweder übereinander in einer Waschsäule oder nebeneinander mit je einer Waschsäule pro Waschstufe angeordnet, so daß sich die Apparatur den jeweiligen räumlichen Begebenheiten anpassen läßt.

Um in der (n-2)ten Waschstufe eine möglichst stöchiometrische Umsetzung von Ammoniak und Schwefelsäure bei gleichzeitig optimaler Wasseraufnahme aus dem Rohgas zu erreichen, ist es zweckmäßig, wenn die Konzentration dar umgewälzten verdünnten Schwefelsäure 75% bis 35%, vorzugsweise 60% bis 45%, beträgt. Ebenso ist es günstig für die $PH_3$-, $H_2S$- und $AsH_3$-Auswaschung in der (n- 1)ten Waschstufe, wenn die Konzentration der umgewälzten konzentrierten Schwefelsäure 95% bis 75%, vorzugsweise 95% bis 85%, beträgt.

Eine besonders gute Feinreinigung des Gases wird erzielt, besonders im Hinblick auf Phosphin ($PH_3$), wenn die Konzentration der frischen konzentrierten Schwefelsäure am Kopf der n-ten Waschstufe mindestens 96 % beträgt.

Mit Vorteil liegt die Gaseintrittstemperatur des Rohgases zwischen 10 und 40°C, insbesondere 15 und 20°C, da die Rohgaseintrittstemperatur ein wesentliches Instrument zur Beeinflussung der Wärmeentwicklung bei der Reaktion von Schwefelsäure mit Wasser aus dem Rohgas darstellt. Somit wird erfindungsgemäß ingesamt eine Temperaturführung in der Kolonne erreicht, die im Hinblick auf die Bildung von Polymerisationsprodukten und die Umsetzung des Phosphorwasserstoffes am günstigsten ist.

Als besonders zweckmäßig beim erfindungsgemäßen Verfahren hat sich die Verwendung von Füllkörperkolonnen erwiesen, da diese einen sehr guten Gas-Flüssigkeitsaustausch gewährleisten und zwischenzeitlich gebildete Ablagerungen problemlos aufgrund der hohen Säureumlaufmenge abgespült werden. Auch ist der Druckabfall in Füllkörperkolonnen vergleichsweise gering, womit die Verwendung von Füllkörperkolonnen insgesamt eine wirtschaftliche und betriebssichere Lösung darstellt.

Weiterhin besteht bei dem erfindungsgemäßen Verfahren die Möglichkeit, den Säurekreislauf zu kühlen und so eine noch effektivere Temperaturkontrolle und -steuerung im Prozeß zu bewirken.

In besonders vorteilhafter Weise wird das erfindungsgemäße Verfahren zur Reinigung von Rohacetylen verwendet. Dies ist von erheblicher Bedeutung, da die Rohacetylenproduktion kontinuierlich erfolgt und mit dem erfindungsgemäßen Verfahren erstmals ein kontinuierliches, betriebssichereres und kostengünstiges Reinigungsverfahren für Rohacetylen, aber auch für Koksofen- oder Wassergas vorgestellt wird, das überdies noch höchste Ansprüche in Bezug auf die Gasreinheit erfüllt.

Des weiteren betrifft die Erfindung eine Vorrichtung zur Auswaschung von Verunreinigungen aus Rohgasen, bestehend aus einer Waschsäule mit Leitungen, die in die Waschsäule münden und der Zu- und Abfuhr von Stoffströmen dienen, sowie mindestens einer Füllkörperkolonne. In kennzeichnender Weise führt eine Leitung für verdünnte Schwefelsäure vom Sumpf der Waschsäule weg und mündet oberhalb einer Rohgaseintrittsleitung wieder in die Waschsäule. Ferner zweigt oberhalb eines im unteren Teil der Waschsäule installierten Kaminbodens eine Leitung für konzentrierte Schwefelsäure ab, die in den oberen Teil der Waschsäule - unterhalb einer Aufgabestelle für frische konzentrierte Schwefelsäure - mündet.

Gemäß einer vorteilhaften Ausgestaltung der erfindungsgemäßen Vorrichtung führt eine Überlaufleitung für konzentrierte Schwefelsäure aus dem mittleren Teil in den unteren Teil der Waschsäule, indem sie mit der Rohgaseintrittsleitung verbunden ist.

Insgesamt bietet die Erfindung den Vorteil, daß, bedingt durch die große Säureumlaufmenge in den Waschstufen 1 und 2, die Säuretemperatur und damit verbunden die Gastemperatur in der Kolonne wesentlich niedriger sind als bei bekannten Verfahren und Vorrichtungen. Dies bewirkt eine wesentlich geringere Bildung von Nebenprodukten. Verlegungen der Waschsäule treten überdies nicht auf, da durch die hohe Säureumlaufmenge Ablagerungen abgespült werden. Somit ist ein kontinuierlicher Betrieb gewährleistet, und die Standzeit der Apparatur ist nahezu unbegrenzt, was die Wirtschaftlichkeit und Betriebssicherheit der Erfindung gegenüber dem Stand der Technik verdeutlicht.

Auch kann auf einen oder mehrere Reservewaschtürme verzichtet werden, was sich günstig auf die Investitionskosten auswirkt.

Ein weiterer Vorteil der Erfindung besteht darin, daß durch die zwei Kreislaufwäschen eine gute Abscheidung von Feststoff-Verunreinigungen, wie sie z.B. bei der Acetylenerzeugung aus Calciumcarbid anfallen, erreicht wird, was im Hinblick auf die Weiterverarbeitung des gereinigten Gases kostengünstig ist.

Überdies ermöglicht es die Erfindung, Säurewaschtürme wesentlich geringerer Höhe (3 - 4 m) einzusetzen, was hinsichtlich Nachrüstbarkeit, Gebäudegröße und zunehmendem Platzmangel eine Verbesserung der Kompaktheit der Anlagen ausmacht.

Nachfolgend sei die Erfindung anhand zweier schematisch dargestellter Ausführungsbeispiele näher erläutert.

Figur 1 Gaswäsche mit Schwefelsäure in drei übereinander angeordneten Stufen.

Figur 2 Gaswäsche mit Schwefelsäure in drei nebeneinander angeordneten Stufen.

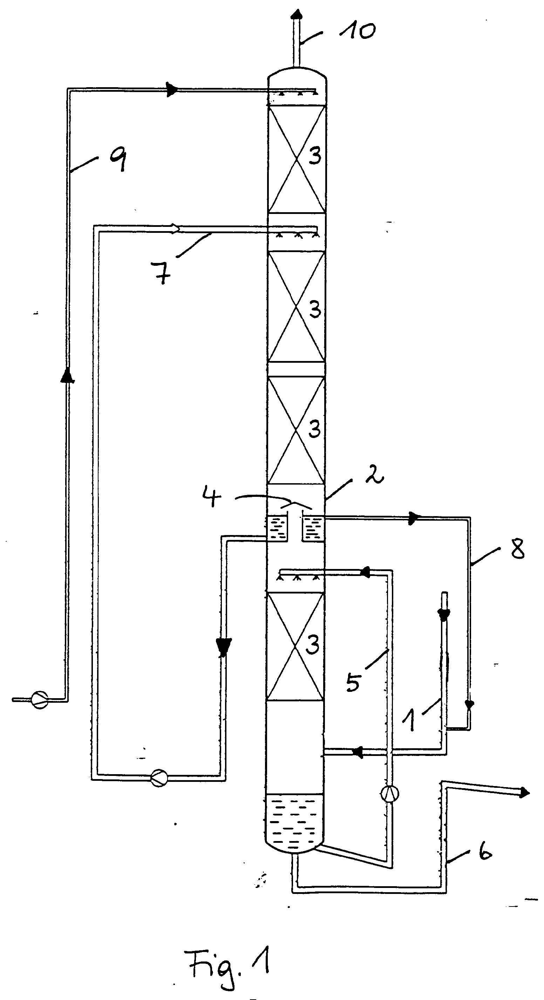

Gemäß Figur 1 wird über Leitung 1 einer Waschsäule 2 ein Rohgasstrom von 400 m³/h folgender Zusammensetzung mit einer Temperatur von 18°C in Sumpfnähe zugeführt :

99,5% $C_2H_2$
500 ppm $PH_3$
200 ppm $H_2S$
50 ppm $AsH_3$
700 ppm $NH_3$
$H_2O$.
gesättigt

Die Waschsäule 2 enthält Füllkörper 3 - z.B. wellblechartig geformte Kolonneneinbauten z.B. aus Kunststoff, die kreuzweise gepackt werden und die zusätzlich noch Löcher aufweisen, so daß ihre sehr große Oberfläche einen geringstmöglichen Druckverlust garantiert - und ist etwa im unteren Drittel der Säule durch einen Kaminboden 4 unterteilt.

Das Rohgas durchströmt die Waschsäule 2 von unten nach oben, wobei es zuerst mit 3 - 4 m³/h umgewälzter verdünnter Schwefelsäure aus Leitung 5 in Kontakt gebracht wird. Dabei wird dem Rohgas das Wasser entzogen und der Ammoniak umgesetzt. Das so entstandene Schwefelsäuregemisch sammelt sich im Säulensumpf, von wo ein Teil mittels der Kreislaufleitung 5 und der andere Teil über Leitung 6 abgezogen wird. Die über Leitung 6 abgezogene verdünnte Schwefelsäure (15 - 20 l/h), die sämtliche löslichen Umsetzungsprodukte enthält, wird beispielsweise einer - nichtdargestellten - Entsorgung zugeführt.

Das nunmehr noch $PH_3$, $H_2S$ und $AsH_3$ als Verunreinigungen enthaltende Rohgas tritt sodann durch den Kaminboden 4 in die zweite Waschstufe ein, wo es mit über Leitung 7 herangeführter konzentrierter umgewälzter Schwefelsäure (5 - 7 m³/h) Kontakt gebracht wird. In diesem Bereich der Waschsäule 2 werden $PH_3$, $H_2S$ und $AsH_3$ aus dem Rohgas zu Phosphorsäure, $SO_2$ und $As_2O_3$ oxidiert bzw. umgesetzt. Das daraus resultierende Schwefelsäuregemisch sammelt sich oberhalb des Kaminbodens und wird in die Kreislaufleitung 7 für konzentrierte Schwefelsäure geführt. Um eine Akkumulation zu vermeiden, ist es notwendig, einen kleinen Teil (8 - 12 l/h) des Schwefelsäuregemisches via Überlaufleitung 8 abzuziehen. Erfindungsgemäß kann diese Leitung in die Rohgasleitung 1 münden. Die überlaufende Schwefelsäure entspricht in der Menge dem Zusatz an Frischsäure. Sie konzentriert den 1. Säurekreislauf auf, der sich durch permanente Wasseraufnahme aus dem Gas verdünnt.

Da in der zweiten Waschstufe nicht der gesamte Phosphorwasserstoff zu Phosphorsäure umgesetzt wird, sondern ein Teil lediglich in der konzentrierten Schwefelsäure in Lösung geht, wird dieses Phosphin beim Wiedereindosieren der konzentrierten Schwefelsäure in die Waschsäule 2 (Leitung 7) freigesetzt (der sogenannte "Boil out") und vermischt sich mit dem aufsteigenden gereinigten Rohgas. In der dritten Waschstufe wird daher dieses nur noch $PH_3$ als Verunreinigung enthaltende Gas mit am Kopf der Waschsäule 2 (Leitung 9) aufgegebener frischer konzentrierter Schwefelsäure (8 - 12 l/h) gewaschen. Somit wird über Leitung 10 ein gereinigtes Gas zur Weiterverwendung abgegeben. Dieses Gas enthält noch 3 - 0 ppm $PH_3$ und als neue Verunreinigung als Restprodukt aus den Oxydationsprozessen 20 - 150 ppm $SO_2$. Das $SO_2$ wird in einem nachfolgenden Waschprozeß z.B. mit verdünnter NaOH entfernt.

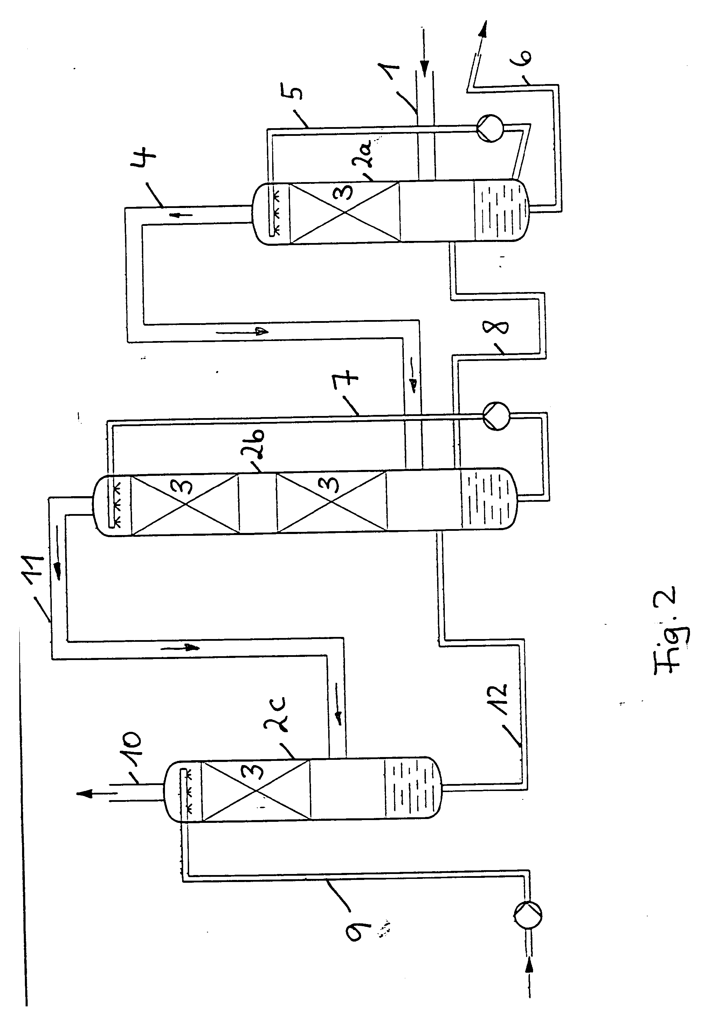

Das Verfahren gemäß Figur 2 ist gleich dem gemäß Figur 1 mit dem Unterschied, daß statt einer einzigen Waschsäule 2 (Fig.1) drei Waschsäulen 2a bis 2c verwendet werden. Gleiche Anlagenteile sind mit denselben Bezugszeichen versehen, wobei statt des Kaminbodens 4 (Fig.1) eine Gasleitung 4 vom Kopf der Säule 2a in den unteren Teil der Säule 2b führt. Die Überlaufleitung 8 mündet hier direkt in den unteren Teil der Waschsäule 2a kurz unterhalb der Rohgasleitung 1 und nicht wie beim Verfahren gemäß Figur 1 z.B. in die Rohgasleitung selbst. Die Verbindung zwischen dem Kopf von Säule 2b und dem unteren Teil der Säule 2c wird mittels der Gasleitung 11 hergestellt. Außerdem führt eine Leitung 12 für konzentrierte Schwefelsäule vom Sumpf der Säule 2c in den unteren Teil der Säule 2b.

In den Ausführungsbeispielen enthaltene Pumpen sind nicht näher bezeichnet.

**Patentansprüche**

1. Verfahren zum Auswaschen von Verunreinigungen wie Phosphorwasserstoff, Ammoniak, Schwefelwasserstoff und/oder Arsenwasserstoff, aus Rohgasen mittels Schwefelsäure in zumindest einer Waschsäule, dadurch gekennzeichnet, daß in einer (n-2)ten Waschstufe, wobei n größer/gleich 3 ist, verdünnte Schwefelsäure und in einer (n-1)ten Waschstufe konzentrierte Schwefelsäure im Kreislauf geführt werden sowie in einer n-ten Waschstufe frische konzentrierte Schwefelsäure am Kopf der Waschsäule aufgegeben wird.

2. Verfahren nach Anspruch 1, dadurch gekennzeichnet, daß vom Sumpf der (n-2)ten Waschstufe überschüssige verdünnte Schwefelsäure abgezogen wird.

3. Verfahren nach Anspruch 1 oder 2, dadurch gekennzeichnet, daß 5 bis 20 l Schwefelsäure pro m³ zu reinigendes Gas im Kreislauf geführt werden.

4. Verfahren nach einem der Ansprüche 1 bis 3, dadurch gekennzeichnet, daß die n Waschstufen übereinander in einer Waschsäule angeordnet werden.

5. Verfahren nach einem der Ansprüche 1 bis 3, dadurch gekennzeichnet, daß die n Waschstufen nebeneinander mit je einer Waschsäule pro Waschstufe angeordnet werden.

6. Verfahren nach einem der Ansprüche 1 bis 5, dadurch gekennzeichnet, daß die Konzentration der verdünnten Schwefelsäure in der (n-2)ten Waschstufe 75% bis 35%, vorzugsweise 60% bis 45%, beträgt.

7. Verfahren nach einem der Ansprüche 1 bis 5, dadurch gekennzeichnet, daß die Konzentration der konzentrierten Schwefelsäure in der (n-1)ten Waschstufe 95% bis 75%, vorzugsweise 95% bis 85%, beträgt.

8. Verfahren nach einem der Ansprüche 1 bis 5, dadurch gekennzeichnet, daß die Konzentration der frischen konzentrierten Schwefelsäure mindestens 96% beträgt.

9. Verfahren nach einem der Ansprüche 1 bis 8, dadurch gekennzeichnet, daß die Gaseintrittstemperatur des Rohgases zwischen 10 und 40°C, insbesondere 15 und 20°C, liegt.

10. Verfahren nach einem der Ansprüche 1 bis 9, dadurch gekennzeichnet, daß als Waschsäulen Füllkörperkolonnen verwendet werden.

11. Anwendung des Verfahrens nach einem der Ansprüche 1 bis 10 zur Reinigung von Rohacetylen.

12. Vorrichtung zur Durchführung des Verfahrens nach Anspruch 1, bestehend aus einer Waschsäule mit Leitungen, die in die Waschsäule münden und der Zu- und Abfuhr von Stoffströmen dienen sowie mindestens einer Füllkörperkolonne, dadurch gekennzeichnet, daß eine Leitung für verdünnte Schwefelsäure vom Sumpf der Waschsäule wegführt und oberhalb einer Rohgaseintrittsleitung wieder in die Waschsäule mündet, sowie daß oberhalb eines im unteren Teil der Waschsäule installierten Kaminbodens eine Leitung für konzentrierte Schwefelsäure abzweigt, die in den oberen Teil der Waschsäule - unterhalb einer Aufgabestelle für frische konzentrierte Schwefelsäure - mündet.

13. Vorrichtung nach Anspruch 12, dadurch gekennzeichnet, daß eine Überlaufleitung für konzentrierte Schwefelsäure aus dem mittleren Teil in den unteren Teil der Waschsäule führt, indem sie mit der Rohgaseintrittsleitung verbunden ist.


**Claims**

1. A process for scrubbing impurities, such as phosphorus hydride, ammonia, hydrogen sulphide and/or arsenic hydride, from crude gases using sulphuric acid in at least one scrubbing column, characterised in that diluted sulphuric acid is circulated in a (n-2)th scrubbing stage, where n is greater than or equal to 3, and concentrated sulphuric acid is circulated in an (n-1)th scrubbing stage, and fresh, concentrated sulphuric acid is added at the head of the scrubbing column in a n-th scrubbing stage.

2. A process as claimed in Claim 1, characterised in that excess, diluted sulphuric acid is discharged from the sump of the (n-2)th scrubbing stage.

3. A process as claimed in Claim 1 or 2, characterised in that 5 to 20 litres of sulphuric acid is circulated per cubic metre of gas which is to be purified.

4. A process as claimed in one of Claims 1 to 3, characterised in that the n scrubbing stages are arranged one above another in a scrubbing column.

5. A process as claimed in one of Claims 1 to 3, characterised in that the n scrubbing stages are arranged one beside another with in each case one scrubbing column per scrubbing stage.

6. A process as claimed in one of Claims 1 to 5, characterised in that the concentration of the diluted sulphuric acid in the (n-2)th scrubbing stage amounts to 75% to 35%, preferably 60% to 45%.

7. A process as claimed in one of Claims 1 to 5, characterised in that the concentration of the concentrated sulphuric acid in the (n-1)th scrubbing stage amounts to 95% to 75%, preferably 95% to 85%.

8. A process as claimed in one of Claims 1 to 5, characterised in that the concentration of the fresh, concentrated sulphuric amounts to at least 96%.

9. A process as claimed in one of Claims 1 to 8, characterised in that the input temperature of the crude gas is between 10 and 40xC, in particular 15 and 20xC.

10. A process as claimed in one of Claims 1 to 9, characterised in that packed columns are used as scrubbing columns.

11. The use of the process claimed in one of Claims 1 to 10 for the purification of crude acetylene.

12. A device for implementing the process claimed in Claim 1, comprising a scrubbing column with pipes which lead into the scrubbing column and serve to supply and discharge arterial stream, and comprising at least one packed column, characterised in that a pipe for diluted sulphuric acid extends from the sump of the scrubbing column and re-enters the scrubbing column above a crude gas input pipe, and that a pipe for concentrated sulphuric acid branches off above a chimney plate installed in the lower part of the scrubbing column, which pipe leads into the upper part of the scrubbing column below a feed station for fresh, concentrated sulphuric acid.

13. A device as claimed in Claim 12, characterised in that an overflow pipe for concentrated sulphuric acid leads from the central part into the lower part of the scrubbing column in that it is connected to the crude gas input pipe.


## Revendications

1. Procédé de lavage d'impuretés tel que hydrogène phosphoré, ammoniac, hydrogène sulfuré et/ou hydrogène arsénié contenues dans un gaz brut au moyen d'acide sulfurique dans au moins une colonne de lavage, caractérisé en ce que dans un (n-2)ème étage de lavage, n étant supérieur ou égal à 3, on introduit de l'acide sulfurique dilué et dans un (n-1)étage de lavage on fait recirculer de l'acide sulfurique concentrée et en ce que dans un n-ème étage de lavage on introduit en tête de la colonne de lavage de l'acide sulfurique concentré frais.

2. Procédé selon la revendication 1, caractérisé en ce que, en cuve du (n-2)ème étage de lavage, on prélève l'acide sulfurique dilué en excès.

3. Procédé selon la revendication 1 ou 2, caractérisé en ce l'on fait circuler de 5 à 20 litres d'acide sulfurique par m3 de gaz à purifier.

4. Procédé selon l'une quelconque des revendications 1 à 3, caractérisé en ce que les n étages de lavage sont disposés l'un au-dessus de l'autre dans une colonne de lavage.

5. Procédé selon l'une quelconque des revendications 1 à 3, caractérisé en ce que les n étages de lavage sont associés chacun à une colonne de lavage dans chaque étage de lavage.

6. Procédé selon l'une quelconque des revendications 1 à 5, caractérisé en ce que la concentration de l'acide sulfurique dans le (n-2)ème étage de lavage est comprise entre 75 et 35%, de préférence 60 et 45%.

7.- Procédé selon l'une quelconque des revendications 1 à 5, caractérisé en ce que la concentration de l'acide sulfurique concentré dans le (n-1)ème étage de lavage est comprise entre 95 et 75%, de préférence 95 et 85%.

8. Procédé selon l'une quelconque des revendications 1 à 5, caractérisé en ce que la concentration de l'acide sulfurique concentré frais est au moins égale à 96%.

9. Procédé selon l'une quelconque des revendications 1 à 8, caractérisé en ce que la température d'entrée du gaz brut est comprise entre 10 et 40°C, notamment 15 et 20°C.

10. Procédé selon l'une quelconque des revendications 1 à 9, caractérisé en ce que l'on utilise comme colonne de lavage des colonnes garnies de corps de remplissage.

11. Application du procédé selon l'une quelconque des revendications 1 à 10 à la purification d'acétylène brut.

12. Dispositif de mise en oeuvre du procédé selon la revendication 1, constitué d'une colonne de lavage comportant des conduites qui débouchent dans la colonne de lavage et qui permettent l'entrée ou la sortie de courants de matières ainsi que d'au moins une colonne garnie de corps de remplissage, caractérisé en ce qu'une conduite d'acide sulfurique dilué part du fond de la colonne de lavage et redébouche dans la colonne de lavage au-dessus d'une conduite d'entrée de gaz brut, et encore en ce que, au-dessus d'un plateau en forme de cloche installé dans la partie inférieure de la colonne de lavage, se trouve une conduite de dérivation d'acide sulfurique concentré qui débouche dans la partie supérieure de la colonne de lavage en-dessous du point d'introduction de l'acide sulfurique concentré frais.

13. Procédé selon la revendication 12, caractérisé en ce qu'une conduite de trop plein d'acide sulfurique concentré passe de la partie médiane à la partie inférieure de la colonne de lavage où elle est raccordée à la conduite d'entrée du gaz brut.

Fig. 1

Fig. 2